# FASCICULE DE BREVET EUROPEEN

(11) **EP 3 573 631 B1**
(45) Date de publication et mention de la délivrance du brevet: **16.11.2022**
(21) Numéro de dépôt: 18702952.5
(22) Date de dépôt: 26.01.2018
(51) Int. Cl.: A61K 35/74, A61P 31/04, A61P 37/08, A61P 35/00, A61P 35/02

(54) **MICROBIOTE FECAL POUR TRAITER DES PATIENTS SUBISSANT UNE GREFFE DE CELLULES SOUCHES HEMATOPOIETIQUES**
FÄKALE MIKROBIOTA ZUR BEHANDLUNG VON PATIENTEN MIT HÄMATOPOIETISCHER STAMMZELLTRANSPLANTATION
FECAL MICROBIOTA FOR TREATING PATIENTS UNDERGOING A HEMATOPOIETIC STEM CELL TRANSPLANT

(30) Priorité: 26.01.2017 FR 1750629
(43) Date de publication de la demande: 04.12.2019
(73) Titulaire: Sorbonne Université, 75006 Paris (FR); Centre National de la Recherche Scientifique, 75016 Paris (FR); Institut National de la Santé et de la Recherche Médicale (INSERM), 75013 Paris (FR); Assistance Publique - Hôpitaux de Paris, 75004 Paris (FR)
(72) Inventeur: MOHTY, Mohamad, 75011 Paris (FR); SOKOL, Harry, 75015 Paris (FR)
(74) Mandataire: Santarelli
(86) Numéro de dépôt international: PCT/EP2018/051941
(87) Numéro de publication internationale: WO 2018/138251

(56) Documents cités:
- EP-A1- 2 922 555
- EP-A2- 0 413 615
- WO-A1-2014/078911
- WO-A1-2016/057671
- ZAMA D ET AL: "Gut microbiota and hematopoietic stem cell transplantation: where do we stand?", BONE MARROW TRANSPLANTATION, vol. 52, no. 1, janvier 2017 (2017-01), pages 7-14, XP055416106,
- KAKIHANA KAZUHIKO ET AL: "Fecal microbiota transplantation for patients with steroid-resistant acute graft-versus-host disease of the gut", BLOOD, vol. 128, no. 16, octobre 2016 (2016-10), pages 2083-2088, XP055402621,
- ISAO TAWARA ET AL: "Influence of Donor Microbiota on the Severity of Experimental Graft-versus-Host-Disease", BIOLOGY OF BLOOD AND MARROW TRANSPLANTATION, vol. 19, no. 1, 1 janvier 2013 (2013-01-01), pages 164-168, XP055252493, US ISSN: 1083-8791, DOI: 10.1016/j.bbmt.2012.09.001
- Timothy J Wilt ET AL: "Evidence-based Synthesis Program Fecal Microbiota Transplantation for Clostridium Difficile Infection: A Systematic Review of the Evidence Co-Investigators: Research Associates", , 1 janvier 2014 (2014-01-01), XP055416020, Extrait de l'Internet: URL:https://www.hsrd.research.va.gov/publi cations/esp/FecalMicrobiota-EXEC.pdf [extrait le 2017-10-16]
- Loebe: "Texas Heart Institute Journal", , 1 janvier 2011 (2011-01-01), XP055416663, Extrait de l'Internet: URL:https://www.ncbi.nlm.nih.gov/pmc/artic les/PMC3231521/pdf/20111000s00018p555.pdf [extrait le 2017-10-18]
- DATABASE BIOSIS [Online] BIOSCIENCES INFORMATION SERVICE, PHILADELPHIA, PA, US; October 2016 (2016-10), KAKIHANA KAZUHIKO ET AL: "Fecal microbiota transplantation for patients with steroid-resistant acute graft-versus-host disease of the gut", Database accession no. PREV201600851167 & BLOOD, vol. 128, no. 16, October 2016 (2016-10), pages 2083-2088, ISSN: 0006-4971(print), DOI: 10.1182/BLOOD-2016-05-717652
- DATABASE BIOSIS [Online] BIOSCIENCES INFORMATION SERVICE, PHILADELPHIA, PA, US; January 2017 (2017-01), ZAMA D ET AL: "Gut microbiota and hematopoietic stem cell transplantation: where do we stand?", Database accession no. PREV201700249166 & BONE MARROW TRANSPLANTATION, vol. 52, no. 1, January 2017 (2017-01), pages 7-14, ISSN: 0268-3369(print), DOI: 10.1038/BMT.2016.173

## Description

L'invention concerne un échantillon de microbiote fécal pour une utilisation dans la prévention d'une maladie GVH résultant d'une greffe allogénique de cellules souches hématopoïétiques (CSH) chez un patient receveur, ledit échantillon de microbiote fécal étant administré avant la greffe de CSH..

### Arrière-plan technologique

Les cellules souches hématopoïétiques sont des cellules à l'origine de toutes les lignées de cellules sanguines. Ces cellules sont capables de donner naissance en se différenciant, à n'importe quelle cellule du sang (globules rouges, globules blancs, plaquettes) et sont aussi capables d'auto- renouvellement.

La greffe de cellules souches hématopoïétiques est un moyen thérapeutique majeur dans le traitement de certaines maladies du sang et de certains cancers. En effet, elle rend possible une intensification thérapeutique par chimiothérapie et/ou de radiothérapie à doses massives ayant pour résultat le traitement de la maladie, voire sa guérison avec une amélioration de la survie du patient. Elle permet aussi l'introduction chez le malade d'un système immunitaire nouveau (cas de l'allogreffe) qui peut contribuer au contrôle de la maladie cancéreuse.

Une greffe effectuée après ces traitements qui comportent une toxicité hématologique importante, permet la reconstruction de la moelle osseuse et le retour à une production normale de cellules sanguines.

La greffe de cellules souches hématopoïétiques intervient dans le traitement de plusieurs types de pathologies : malignes comme les leucémies aiguës, les myélomes, les lymphomes ou certaines tumeurs solides (cancer du sein, cancer du testicule, cancer de l'ovaire, neuroblastome, etc.) mais aussi non malignes comme les déficits constitutionnels ou acquis (déficit immunitaire, aplasies, déficits et erreurs métaboliques etc.).

Deux types de greffe peuvent être pratiqués :
- l'autogreffe où le patient reçoit ses propres cellules souches prélevées dans la moelle osseuse, ou dans le sang périphérique plusieurs semaines, mois ou années auparavant et conservées congelées.
- l'allogreffe (ou « greffe allogénique ») qui nécessite un donneur familial compatible ou pas, ou non apparenté compatible ou pas, ou des cellules de sang de cordon ombilical.

La greffe s'effectue selon plusieurs étapes. Quelques jours avant de subir la greffe, le patient est typiquement soumis à un « conditionnement » myéloablatif ou non myéloablatif. Il s'agit souvent d'une chimiothérapie plus ou moins forte et éventuellement une irradiation totale pour détruire la moelle du receveur et permettre la prise de greffe. En général, le but est de détruire toutes les cellules présentes dans la moelle osseuse du receveur pour permettre aux cellules souches hématopoïétiques du donneur de s'y développer et se substituer ainsi à la moelle osseuse détruite du receveur.

La greffe de cellules hématopoïétiques s'effectue ensuite par transfusion intraveineuse. La greffe est suivie par une période d'aplasie pendant laquelle le patient n'a plus de défenses immunitaires.

Dans le cas des allogreffes, la période de « conditionnement » précédent la greffe allogénique permet de créer une immunosuppression suffisante pour empêcher le rejet du greffon, et le cas échéant obtenir un effet anti-tumoral sur les cellules malignes. Toutefois, cet effet bénéfique est contrebalancé par les risques élevés de maladie du greffon contre l'hôte (GVH : Graft versus host) liée aux réactions immunologiques entre le donneur et le receveur. Par ailleurs, le « conditionnement » expose le patient à des risques d'infections bactériennes, virales ou fongiques, nécessitant la mise en place de traitements anti-infectieux prophylactiques ou curatifs.

A ce jour, aucun traitement de ces complications graves n'est entièrement satisfaisant.

Il existe toujours un besoin en une approche préventive ou curative de telles complications.

### Résumé de l'invention

En réponse à ce besoin thérapeutique, les inventeurs proposent de combiner la greffe de CSH à une transplantation de microbiote fécal. Les inventeurs ont plus particulièrement identifié l'intérêt que le donneur de microbiote fécal soit le même que le donneur de CSH. Ils ont également identifié l'intérêt d'une transplantation de microbiote fécal avant la greffe de CSH, que ce soit dans le cas d'une situation isologue (où donneur de microbiote fécal et donneur de CSH sont identiques) ou dans le cas d'une situation où donneur de microbiote fécal et donneur de CSH sont différents.

L'invention est telle que définie dans les revendications 1 à 7.

L'invention concerne ainsi un échantillon de microbiote fécal pour une utilisation dans la prévention d'une maladie GVH résultant d'une greffe allogénique de cellules souches hématopoïétiques (CSH) chez un patient receveur, ledit échantillon de microbiote fécal étant administré avant la greffe de CSH.

Il est également décrit (mode de réalisation non compris dans l'invention revendiquée) un échantillon de microbiote fécal pour son utilisation dans la prévention et/ou le traitement de complications infectieuses ou non-infectieuses résultant d'une greffe allogénique de CSH chez un patient receveur, ledit échantillon de microbiote fécal et lesdites CSH provenant d'un même sujet donneur.

La transplantation de microbiote fécal peut être réalisée avant ou après l'allogreffe de CSH (mode de réalisation non compris dans l'invention revendiquée).

Il est également décrit (mode de réalisation non compris dans l'invention revendiquée) un échantillon de microbiote fécal pour son utilisation dans la prévention et/ou le traitement de complications infectieuses ou non-infectieuses résultant d'une greffe allogénique de CSH chez un patient receveur, ledit échantillon de microbiote fécal étant administré avant la greffe de CSH.

Avantageusement, le microbiote fécal permet d'empêcher la survenue, ou de réduire le risque de survenue, d'une maladie du greffon contre l'hôte (GVH) suite à la greffe allogénique de CSH.

Le microbiote fécal permet en outre de réduire le risque d'une complication infectieuse, pouvant aller jusqu'à la septicémie, qui serait due à une colonisation de bactéries pathogènes, notamment de bactéries multi-résistantes aux antibiotiques, ayant infecté le patient receveur avant la greffe allogénique de CSH, et avant même le démarrage du conditionnement myéloablatif ou non myéloablatif du patient. Une telle infection est typiquement une contre-indication à la greffe allogénique de CSH. La transplantation de microbiote fécal selon l'invention résout cette contre-indication, le patient devenant apte à recevoir un conditionnement et une greffe allogénique de CSH.

Le patient receveur peut en particulier souffrir d'un cancer, notamment une hémopathie maligne, mais aussi d'une maladie non maligne.

Il est également décrit (mode de réalisation non compris dans l'invention revendiquée) un échantillon de microbiote fécal pour son utilisation dans le traitement d'un cancer chez un patient receveur souffrant d'un cancer, en particulier d'une hémopathie maligne, et subissant ou ayant subi une greffe allogénique de CSH, ledit échantillon de microbiote fécal et lesdites CSH provenant de préférence d'un même sujet donneur.

Là encore la transplantation de microbiote fécal peut être réalisée avant ou après la greffe de CSH.

### Description détaillée de l'invention

### Le patient receveur

Le patient visé est un humain, quel que soit son âge et son sexe, qui va subir une greffe de cellules souches hématopoïétiques. Le patient soufre de toute maladie pouvant être traitée par une greffe de CSH. Il s'agit notamment de cancers, de maladies génétiques, de maladies qui affectent le système hématopoïétique et/ou le système immunitaire. On peut notamment citer : les tumeurs solides, les hémopathies malignes, dont la leucémie myéloïde chronique, la leucémie myéloïde aiguë, la leucémie lymphoblastique aiguë, les syndromes myéloprolifératifs, les syndromes myélodysplasiques, les LNH (lymphomes malins non hodgkiniens), la maladie de Hodgkin, le myélome multiple, l'aplasie médullaire idiopathique, l'hémoglobinurie nocturne paroxystique, les hémoglobinopathies, les déficits immunitaires congénitaux (SCID, Kostmann, Wiskott-Aldrich) et l'anémie de Fanconi.

Le patient subit un conditionnement (ou préparation) avant la greffe de cellules souches hématopoïétiques, qui peut être un traitement myéloablatif ou non myéloablatif.

Une revue de ce type de traitement est présentée dans Petersen et al, 2007 (Alloreactivity of therapeutic principle in the treatment of hematologic malignancies . Danish Médical Bulletin, May 2007, 54 :112-39). Un exemple de traitement myéloablatif peut être le suivant : la cyclophosphamide (120mg/kg) combinée avec du busulfan (16mg/kg) ou avec une irradiation totale du patient avec une dose de rayon absorbée de 12 Gy (« total body irradiation »).

Dans le cas du traitement non-myéloablatif, le patient peut être totalement irradié avec dose de radiation inférieure à 12Gy ou recevoir une chimiothérapie à base de cyclophosphamide et/ou fludarabine. Par exemple on peut prévoir un traitement lymphopéniant non myéloablatif, qui comprend une injection intraveineuse de cyclophosphamide 50mg/kg une fois à J-6 et J-5, et injection de fludarabine 25mg/m² de J-6 à J-2 (J0 étant le jour de la greffe de cellules souches hématopoïétiques). Cf Sandmaier et al, Semin Oncol. 2000 Apr;27(2 Suppl 5):78-8.

Un conditionnement séquentiel avec du Thiotepa est également possible, tel que décrit dans Duléry et al, Biol Blood Marrow Transplant. 2018 Jan 11. pii: S1083-8791(18)).

Les cellules souches hématopoïétiques utilisées peuvent être issues de n'importe quelle source, par exemple du sang périphérique ou de la moelle osseuse, d'un sujet donneur.

### Le microbiote fécal

L'invention met en œuvre une transplantation d'échantillon de microbiote fécal pouvant avantageusement provenir selon certains modes de réalisation, du même sujet donneur que le sujet donneur de CSH.

Par « microbiote fécal », on entend la flore microbienne, notamment bactérienne, présente dans les selles d'un individu sain. Le microbiote intestinal humain est l'ensemble des micro-organismes (bactéries, levures et champignons) qui se trouvent dans le système gastro-intestinal humain (estomac, intestin et côlon). La diversité microbienne est estimée à l'heure actuelle à environ 10 espèces bactériennes composant le microbiote intestinal dominant d'un individu adulte, avec une abondance de 1014 bactéries, représentant un métagénome bactérien de 200 000 à 800 000 gènes chez chaque individu, soit 10 à 50 fois le nombre de gènes du génome humain. Stérile *in utero,* l'intestin se colonise dès les premiers jours de vie jusqu'à évoluer vers un microbiote individuel unique. Chaque personne possède des bactéries relativement proches en termes d'espèces, mais la composition exacte de son microbiote (espèces, proportions) est pour une large part (plus de ¾ des espèces) spécifique de l'hôte.

Par « transplantation de microbiote fécal » ou « transplantation d'un échantillon de microbiote fécal », on entend l'administration de la flore microbienne, notamment bactérienne, fécale d'un individu sain chez un patient.

Dans le contexte de la présente invention, le « patient » receveur de la greffe de cellules souches hématopoïétiques est distinct du « sujet » donneur des cellules souches hématopoïétiques et du « sujet » donneur de l'échantillon de microbiote fécal, étant entendu que, dans certains modes de réalisation particuliers, «sujet» donneur des cellules souches hématopoïétiques est également «sujet» donneur de l'échantillon de microbiote fécal. Le «sujet» donneur de cellules souches hématopoïétiques, humain, présente de préférence une bonne histocompatibilité avec le « patient » receveur, à savoir que le donneur a un typage HLA le plus proche possible du receveur. On préfère ainsi qu'au moins 5 à 10 antigènes HLA soient compatibles.

De préférence, le sujet donneur de microbiote fécal est un sujet sain. Par sujet « sain », on entend un sujet non atteint d'un déséquilibre du microbiote intestinal ou d'une pathologie diagnostiquée/reconnue par le corps médical. De préférence encore, le sujet donneur de microbiote fécal est un proche du patient, de préférence un membre de sa famille, vivant dans le même environnement.

L'échantillon de microbiote fécal comprend typiquement des bactéries du genre Blautia, Ruminococcus, Eubacterium, Holdemania, et Clostridium, notamment les bactéries des espèces Ruminococcus obeum, Clostridium hathewayi, Eubacterium desmolans, Dorea longicatena, Ruminococcus lactaris (Blautia producta), Eubacterium contorum, Ruminococcus faecis, Holdemania filiformis, Clostridium sordelli.

L'échantillon de microbiote fécal peut être obtenu et conservé par tout moyen connu de l'homme du métier, tout en veillant avantageusement à préserver la viabilité des bactéries qui composent le microbiote fécal, en particulier les bactéries anaérobies.

Cette étape se fait de préférence par prélèvement d'un échantillon de selles du sujet donneur. En effet, l'échantillon de selles contient du microbiote fécal du sujet donneur. Ainsi, le procédé comprend une étape de prélèvement d'au moins un échantillon de selles, comprenant le microbiote fécal, du sujet donneur.

De préférence, l'échantillon de selles présente une masse d'au moins 10g.

L'échantillon de microbiote fécal peut être congelé, pour une utilisation à l'état décongelé, ou être préparé à partir de selles fraîches. Un mélange de selles fraîches et d'échantillon congelé est également possible.

Selon un mode de réalisation particulier, on utilise dans l'invention un échantillon susceptible d'être obtenu par décongélation à partir d'un i) mélange d'un échantillon de selles du sujet donneur avec une solution aqueuse saline comprenant un agent cryoprotectant et/ou un agent de charge, suivi éventuellement d'une ii) filtration, avant iii) congélation pour conservation, les étapes de mélange et de congélation étant de préférence réalisées dans des conditions d'anaérobiose.

Un tel procédé de préparation d'un échantillon de microbiote fécal est par exemple décrit dans la demande de brevet WO2016/170285.

Il peut notamment comprendre les étapes suivantes:
a) le prélèvement d'au moins un échantillon de microbiote fécal du sujet donneur,
b) le placement dudit échantillon obtenu en a) dans un dispositif de collecte, de préférence étanche à l'oxygène, de préférence dans un délai inférieur à 5 minutes suivant le prélèvement,
c) le mélange de l'échantillon obtenu en b) avec au moins une solution aqueuse saline comprenant au moins un cryoprotectant et/ou un agent de charge,
d) optionnellement, la filtration du mélange obtenu en c), notamment par un filtre comprenant des pores de diamètre inférieur ou égal à 0.7 mm, de préférence inférieur ou égal à 0.5 mm et
e) le stockage du mélange obtenu en c) ou d) par congélation à une température typiquement comprise entre -15°C et -100°C, de préférence entre -60°C et -90°C,
les étapes b) à e) étant de préférence réalisées en anaérobiose.

Une fois l'échantillon (obtenu en a) placé dans un dispositif de collecte, de préférence étanche à l'oxygène, il peut, de façon optionnelle, être incubé à une température comprise entre 33°C et 40°C pendant une durée maximale de 75h. De préférence, cette étape d'incubation se fait à une température comprise entre 35°C et 38°C, pendant une durée comprise entre 24h et 73h. Idéalement, cette étape se fait à une température d'environ 37°C pendant 72h. Alternativement, l'échantillon peut, de façon optionnelle, être incubé à une température comprise entre 2°C et 10°C pendant une durée maximale de 75h. De préférence, cette étape d'incubation se fait à une température comprise entre 4°C et 8°C, pendant une durée comprise entre 24h et 72h.

Puis intervient l'étape c) : cette étape comprend le mélange de l'échantillon obtenu en b) avec au moins une solution aqueuse saline comprenant au moins un cryoprotectant et/ou un agent de charge.

Typiquement, la solution aqueuse saline comprend de l'eau et des sels physiologiquement acceptables. Typiquement, les sels sont des sels de calcium, sodium, potassium ou magnésium, avec des ions chlorure, gluconate, acétate ou hydrogénocarbonate.

La solution aqueuse saline peut également optionnellement comprendre au moins un antioxydant. L'antioxydant est notamment choisi parmi l'acide ascorbique et ses sels (ascorbate), les tocophérols (notamment -tocophérol), la cystéine et ses formes salifiées (chlorhydrate notamment) et leurs mélanges.

De préférence, la solution aqueuse saline comprend :
- au moins un sel choisi parmi le chlorure de sodium, le chlorure de calcium, le chlorure de magnésium, le chlorure de potassium, le sodium gluconate et le sodium acétate, et
- optionnellement au moins un antioxydant, de préférence choisi parmi le L-ascorbate de sodium, les tocophérols, le chlorhydrate de L-cystéine monohydraté et leurs mélanges. Typiquement, le sel est présent dans la solution aqueuse saline en concentration comprise entre 5 et 20g/L, de préférence entre 7 et 10 g/L.

Typiquement, l'antioxydant est présent dans la solution aqueuse saline en quantité comprise entre 0.3 et 1% en poids par rapport au volume total de solution, de préférence en quantité comprise entre 0.4 et 0.6% en poids par rapport au volume total de solution. De préférence, lorsque l' antioxydant est un mélange de L-ascorbate de sodium et de chlorhydrate de L-cystéine monohydraté, le L-ascorbate de sodium est présent en une quantité comprise entre 0.4 et 0.6% en poids par rapport au volume total de solution, et le chlorhydrate de L-cystéine monohydraté est présent en une quantité comprise entre 0.01 et 0.1% en poids par rapport au volume total de solution.

De préférence, la solution aqueuse saline comprend également au moins un cryoprotectant. Un cryoprotectant est une substance utilisée pour protéger l'échantillon des dommages causés par la congélation, notamment dus à la formation de cristaux de glace.

De préférence, le cryoprotectant est choisi parmi les polyols, les di- à pentasaccharides (les disaccharides, les trisaccharides, les quadrisaccharides et les pentasaccharides), le DMSO et leurs mélanges. De préférence, le cryoprotectant est choisi parmi les polyols, les tri- et disaccharides, le DMSO et leurs mélanges. Plus préférentiellement, le cryoprotectant présent dans la solution aqueuse saline est un disaccharide ou un trisaccharide.

Parmi les polyols utilisables, on trouve notamment le glycérol, le mannitol, le sorbitol, mais également le propylène glycol ou l'éthylène glycol. Parmi les di- à pentasaccharides utilisables, on peut citer les dimères, trimères, quadrimères et pentamères d'unités identiques ou différentes, lesdites unités étant choisies parmi le glucose, le fructose, le galactose, le fucose et l'acide N-acétylneuraminique. Parmi les disaccharides utilisables, on trouve notamment le tréhalose ou l'un de ses analogues, ou le saccharose. Enfin, le DMSO, ou diméthylsulfoxide, est un cryoprotectant classique.

Ces cryoprotectants peuvent être utilisés seuls ou en mélange.

Typiquement, la quantité totale de cryoprotectant présent dans la solution aqueuse saline est comprise entre 3 et 30% en poids par rapport au volume total de solution, de préférence entre 4% et 20% en poids par rapport au volume total de solution.

De préférence, le cryoprotectant est choisi parmi le glycérol, le mannitol, le sorbitol, le DMSO, le propylène glycol, l'éthylène glycol, le tréhalose et ses analogues, le saccharose, le galactose-lactose et leurs mélanges. Plus préférentiellement, le cryoprotectant est le galactose-lactose ou le tréhalose.

De préférence, la solution aqueuse saline comprend au moins un agent de charge.

L'agent de charge est de préférence choisi parmi les hydrolysats partiels d'amidon ou de fécule. Les hydrolysats partiels d'amidon, notamment de blé ou de maïs, ainsi que les hydrolysats partiels de fécule, par exemple de pomme de terre, comprennent une forte quantité de maltodextrines. Les maltodextrines sont le résultat de l'hydrolyse partielle d'amidon ou de fécule, et sont constituées de différents sucres (glucose, maltose, maltotriose, oligo- et polysaccharides), dont les proportions varient en fonction du degré d'hydrolyse.

De préférence, l'agent de charge présent dans la solution aqueuse saline est un mélange de maltodextrines, dans lequel la quantité de maltodextrines est comprise entre 4 et 20% en poids par rapport au volume total de solution.

De préférence, la solution aqueuse saline comprend à la fois :
- au moins un cryoprotectant tel que décrit ci-dessus, i.e. choisi parmi les polyols, les di-à pentasaccharides (i.e. les disaccharides, les trisaccharides, les quadrisaccharides et les pentasaccharides), le DMSO et leurs mélanges, et
- au moins un agent de charge tel que décrit ci-dessus, i.e. choisi parmi les hydrolysats partiels d'amidon ou de fécule, de préférence l'agent de charge est constitué de maltodextrines.

De préférence, dans ce cas, la quantité de cryoprotectant est comprise entre 3 et 30% en poids par rapport au volume total de solution, de préférence entre 4% et 20% en poids par rapport au volume total de solution ; et la quantité d'agent de charge, de préférence de maltodextrines, est comprise entre 4 et 20% en poids par rapport au volume total de solution.

L'étape c) de mélange de l'échantillon obtenu en b) avec au moins une solution aqueuse saline comprenant au moins un cryoprotectant peut notamment être réalisée par malaxage, afin d'obtenir un mélange homogène.

De préférence, l'échantillon obtenu en b) est mélangé avec ladite solution aqueuse saline dans un rapport poids/volume respectif compris entre 0.5 poids : 10 volumes et 2 poids : 2 volumes. Un rapport poids/volume échantillon : solution égal à 0.5 poids : 10 volumes signifie que l'échantillon est mélangé à 0.5 poids (par exemple 0.5g) pour 10 volumes de solution (par exemple 10ml). De préférence, le rapport poids/volume échantillon : solution est égal à 1 poids d'échantillon pour 4 volumes de solution (1 poids : 4 volumes).

Une fois cette étape réalisée, une étape d) optionnelle peut être effectuée. L'étape d) comprend la filtration du mélange obtenu en c), notamment par un filtre comprenant des pores de diamètre inférieur ou égal à 0.7 mm, de préférence inférieur ou égal à 0.5 mm. Une telle filtration permet la rétention des particules grossières, et la récupération des bactéries d'intérêt (constitutives du microbiote fécal) dans le filtrat.

Puis, suite à l'étape c) ou d), lorsque cette dernière a lieu, le mélange obtenu est stocké par congélation à une température comprise entre -15°C et -100°C : c'est l'étape e). De préférence, la température de congélation (et donc de stockage) est comprise entre -60°C et -90°C ; plus préférentiellement elle est d'environ -80°C ou d'environ -65°C.

Afin d'être congelé, suite à l'étape c) ou d), et avant l'étape e), le mélange peut être préalablement aliquoté, pour assurer des spécimens de volume constant. Par exemple, l'aliquotage est effectué pour obtenir des spécimens de volume égal à 50 ml, 100 ml, 150 ml, ou 200 ml. De préférence, l'aliquotage est effectué pour obtenir des spécimens de volume égal à 100 ml.

Cette étape de congélation et stockage permet de conserver les échantillons traités pendant une durée d'au moins 2 mois. Les échantillons ainsi stockés présentent en outre une bonne qualité, même après décongélation.

De préférence, le procédé comprend une étape f) de décongélation de l'échantillon congelé obtenu en e), en anaérobiose, jusqu'à température ambiante. Cette étape f) de décongélation peut s'effectuer en mettant l'échantillon congelé au bain-marie à une température comprise entre 35°C et 40°C, par exemple 37°C, pendant une durée de quelques minutes (typiquement de 2 à 10 minutes). L'étape f) de décongélation peut également s'effectuer en mettant l'échantillon congelé à une température comprise entre 2°C et 10°C, par exemple entre 4°C et 8°C, pendant une durée de 10 à 20 heures.

L'échantillon ainsi décongelé, à température ambiante, peut ensuite être administré au patient receveur.

Dans un exemple de réalisation préféré, les selles sont manipulées dans un poste de sécurité microbiologique dans les 6 heures après émission. Les selles du donneur sont pesées et mélangé avec une solution saline de cryopreservation (glycerol + NaCl 0.9% ,10/90 V/V) en utilisant une balance, un blender, des récipients stériles et du matériel médical stérile (seringues, filtres...). L'homogénéisation se fait dans 500ml pour 50-100g de selles. La suspension obtenue est filtrée en utilisant un entonnoir avec des compresses stériles et des récipients stériles pour retirer tout résidu solide avant la congélation à -80°C.

Le jour précédant la transplantation fécale, la solution congelée est placée au réfrigérateur (entre 4 et 8°C) pour une décongélation pendant la nuit. Le jour de la transplantation fécale, la solution est placée dans un sac à lavement. Le matériel est prêt à être utilisé.

Dans un autre mode de réalisation, on peut utiliser des selles fraîches, à savoir non congelées. Dans un exemple de réalisation préféré, les selles sont manipulées dans un poste de sécurité microbiologique dans les 6 heures après émission. Les selles du donneur sont pesées et mélangé avec une solution saline stérile (NaCl 0.9% ,10/90 V/V) en utilisant une balance, un blender, des récipients stériles et du matériel médical stérile (seringues, filtres...). L'homogénéisation se fait dans 500ml pour 50-100g de selles. La suspension obtenue est filtrée en utilisant un entonnoir avec des compresses stériles et des récipients stériles pour retirer tout résidu solide. La solution est ensuite placée dans un sac à lavement. Le matériel est prêt à être utilisé.

### Indications thérapeutiques

Il est décrit ici (mode de réalisation non compris dans l'invention revendiquée) un échantillon de microbiote fécal pour son utilisation dans la prévention et/ou le traitement de complications infectieuses ou non-infectieuses résultant d'une greffe allogénique de cellules souches hématopoïétiques (CSH) chez un patient receveur, comprenant l'administration audit patient receveur d'un échantillon de microbiote fécal, ledit échantillon de microbiote fécal et lesdites cellules souches hématopoïétiques provenant de préférence d'un même sujet donneur. Par « complications infectieuses ou non-infectieuses résultant d'une greffe allogénique de CSH », on entend notamment les complications dues au conditionnement du patient pour la greffe allogénique de CSH, conditionnement qui réduit les défenses immunitaires du patient, ainsi que les complications dues à la greffe allogénique elle-même, à savoir notamment une maladie du greffon contre l'hôte (GVH) dans ses formes aigues et/ou chroniques.

Il est également décrit (mode de réalisation non compris dans l'invention revendiquée) un échantillon de microbiote fécal pour son utilisation dans une méthode de traitement par greffe de cellules souches hématopoïétiques (CSH) d'un patient qui a besoin d'un tel traitement, dans laquelle on administre audit patient, avant et/ou après la greffe de CSH, une quantité appropriée d'échantillon de microbiote fécal, ledit échantillon de microbiote fécal provenant de préférence d'un même individu donneur que les CSH.

Le terme « traitement » inclut un traitement curatif aboutissant à une guérison ou un traitement allégeant, améliorant, éliminant, réduisant et/ou stabilisant les symptômes d'une maladie ou la souffrance qu'elle provoque.

Le terme « prévention » correspond à un traitement prophylactique ou préventif, qui comprend aussi bien un traitement aboutissant à la prévention d'une maladie qu'un traitement réduisant et/ou retardant l'incidence d'une maladie ou le risque qu'elle survienne.

La transplantation d'un échantillon fécal diminue le risque pour le patient receveur, de développer une ou plusieurs complications infectieuses ou non-infectieuses suite à la greffe de CSH, et/ou améliore ou guérit une ou des complications qui surviennent après la greffe de CSH. Le fait que l'échantillon de microbiote fécal provient du même donneur que les CSH permettent une meilleure tolérance de la transplantation de microbiote fécal (TMF) par le patient, dans le cas où la TMF a lieu après la greffe de CSH. En effet, lors de la greffe préalable de CSH, les cellules immunitaires du donneur sont transférées dans le patient receveur. Les réactions immunitaires lors de la TMF sont alors réduites du fait de la présence de cellules immunitaires de ce même donneur dans le corps du patient receveur.

Une TMF réalisée avant la greffe de CSH permet de réduire, voire éliminer, les infections par des bactéries résistantes, par exemple *Citrobacter freundii* ou *Pseudomonas aeruginosa,* dont serait porteur le patient candidat, avant la greffe de CSH et son conditionnement préalable. Elle permet en outre une correction du microbiote intestinal du patient candidat à la greffe de CSH, et par conséquent permet de diminuer l'incidence des complications pouvant survenir après la greffe de CSH.

L'invention permet également de diminuer les cures d'antibiothérapies vis-à-vis des infections. Les complications infectieuses sont notamment une infection par Clostridium ou par entérocoques, notamment d'une infection par entérocoques résistants à la vancomycine (ERV) ou toute autre germe résistant aux antibiotiques classiques.

Les infections à *Clostridium difficile,* notamment les infections récidivantes, sont particulièrement visées. Parmi les autres espèces opportunistes qui peuvent provoquer des complications infectieuses, on peut citer *E. faecalis, P. mirabilis,* ou *E. coli.*

La complication non-infectieuse la plus connue est la maladie du greffon-contre-l'hôte (GVH). La GVH aigue survient généralement dans les 6 mois qui suivent la greffe de CSH et peut avoir une fréquence comprise entre 10 et 80% des cas selon la disparité génétique entre donneur et receveur. On parle généralement de GVH chronique quand elle intervient plus de 100 jours après la greffe ou encore en fonction de certaines caractéristiques cliniques de cette GVH

D'autres complications incluent les entéropathies et autres complications digestives post-greffe.

Il est également décrit (mode de réalisation non compris dans l'invention revendiquée) un échantillon de microbiote fécal pour son utilisation dans le traitement du cancer, chez un patient receveur souffrant d'un cancer et subissant ou ayant subi une greffe allogénique de cellules souches hématopoïétiques, l'échantillon de microbiote fécal étant administré audit patient, ledit échantillon de microbiote fécal et lesdites cellules souches hématopoïétiques provenant de préférence d'un même sujet donneur.

Le terme « traiter » ou « traitement » inclut un ralentissement de la progression du cancer, une rémission complète, ou une diminution du risque de rechute.

Il peut notamment s'agir de traiter une tumeur solide ou une hémopathie maligne, comme citées plus haut.

En effet, la transplantation de microbiote fécal et la greffe de CSH du même donneur peuvent offrir un effet synergique vis-à-vis de l'élimination des cellules cancéreuses résiduelles, et donc la rémission persistante du cancer. De fait, les CSH contiennent des cellules immunocompétentes (cellules Natural Killer, lymphocytes B et T en particulier), qui ont déjà été en contact avec l'environnement du donneur. Ces CSH contiennent donc des lymphocytes et autres effecteurs immuns spécifiques pré-activés par les antigènes des bactéries commensales (de la flore intestinale) du donneur. Sans être liés par un mécanisme d'action particulier, les inventeurs font l'hypothèse que ces effecteurs immuns pré-activés transplantés dans le receveur sont restimulés par les bactéries transplantées.

Il est aussi décrit ici (mode de réalisation non compris dans l'invention revendiquée) un produit de combinaison (« kit of parts ») comprenant d'une part des cellules souches hématopoïétiques humaines (CSH) et d'autre part un échantillon de microbiote fécal, les CSH et l'échantillon de microbiote fécal proviennent de préférence d'un même individu donneur, pour une utilisation combinée simultanée ou décalée dans le temps dans un traitement par greffe de CSH, tel que défini précédemment.

Un kit peut être aussi fourni, comprenant d'une part des cellules souches hématopoïétiques humaines (CSH) dans un conditionnement approprié et d'autre part un échantillon de microbiote fécal dans un conditionnement approprié, les CSH et l'échantillon de microbiote fécal provenant de préférence d'un même individu donneur. Les méthodes de prélèvement et de conditionnement des CSH d'une part et des échantillons de microbiote fécal d'autre part sont bien connues de l'homme du métier, et notamment décrites ci-dessus. Par « kit », on entend que les deux éléments qui le constituent sont associés pour un usage combiné, et qu'ils peuvent être conservés ensemble ou séparément pour leur utilisation chez un même patient.

### Protocoles

L'échantillon de microbiote fécal peut être administré (on dit aussi « transplanté ») dans le tube digestif du patient receveur avant ou après ladite greffe de CSH (mode de réalisation non compris dans l'invention revendiquée).

Le patient peut recevoir une administration unique de l'échantillon de microbiote fécal, ou des administrations répétées, par exemple de 4 à 8 jours ou de 2 à 8 semaines d'écart.

Il est décrit (mode de réalisation non compris dans l'invention revendiquée) que l'échantillon de microbiote fécal peut être transplanté dans le tube digestif du patient receveur de 1 semaine à 2 ans, de préférence de 1 semaine à 1 an, de préférence encore de 1 semaine à 6 mois, et encore de préférence de 1 semaine à 3 mois, après ladite greffe de CSH, éventuellement de manière répétée.

Dans un mode de réalisation particulier, l'échantillon de microbiote fécal est transplanté dans le tube digestif du patient receveur de 1 semaine à 12 mois, de préférence 1 semaine à 8 mois, de préférence 1 semaine à 6 mois, de préférence encore de 1 semaine à 4 mois, de préférence 1 semaine à 3 mois, ou de 1 à 8 semaines, de préférence de 1 à 6 semaines, avant ladite greffe de CSH, éventuellement de manière répétée.

De manière générale, les quantités administrées sont telles qu'elles permettent la colonisation du tractus digestif par les bactéries et autres micro-organismes du microbiote fécal implanté. Les quantités administrées sont typiquement comprises entre 100 ml et 1L, de préférence environ 500ml, de solution saline préparée à partir d'un échantillon fécal, comme décrit plus haut.

De préférence l'échantillon de microbiote fécal comprend 10¹⁰ à 10¹³ bactéries/g d'échantillon. La transplantation de l'échantillon de microbiote fécal peut être réalisée par une administration par voie rectale, par voie orale (par exemple sous forme lyophilisée, ou sous forme de gélules), ou par toute méthode permettant le transfert du microbiote dans le tractus intestinal. Par exemple des poches à lavement peuvent être employées à cette fin. La transplantation de l'échantillon de microbiote fécal peut également être réalisée au moyen d'un colonoscope, appareil qui sert habituellement à examiner le côlon.

Les exemples suivants illustrent l'invention sans en limiter la portée.

### Exemple 1 : Transplantation de microbiote fécal isologue post-greffe de CSH, chez une patiente atteinte de leucémie myéloïde aigüe (mode de réalisation non compris dans l'invention revendiquée)

### Protocole :

La patiente est une jeune fille de 16 ans atteinte de leucémie myéloïde aigüe, en récidive après une première allogreffe de CSH.

Cette patiente a reçu une seconde greffe de CSH provenant de sa mère, après un conditionnement d'intensité réduite incluant une combinaison de médicaments de chimiothérapie. La patiente a reçu 5mg/kg de Thiotepa six jours avant la greffe, puis, entre cinq et deux jours avant la greffe, de la fludarabine 40 mg/m²/jour, et, entre 5 et 4 jours avant la greffe, du busulfan à 3,2 mg/kg/jour. Enfin, entre trois et cinq jours après la greffe la patiente a reçu 50mg/kg/jour de cyclophosphamide.

La patiente n'a pas développé de réaction aigüe du greffon contre l'hôte dans les suites immédiates de la greffe et son traitement immunosuppresseur à base de ciclosporine-A et de mycophénolate mofetil a pu être arrêté 83 jours après la greffe.

En revanche de multiples complications bactériennes (sepsis, bactéries multi-résistantes) ont été observées durant les premiers mois post-greffe, nécessitant l'utilisation d'antibiotiques à large spectre. Un mois après la greffe, la patiente a développé un premier épisode d'infection à *Clostridium difficile* qui a été traité avec succès par metronidazole. Deux mois et demi après la greffe, la présence d'entérocoques résistants à la vancomycine (ERV) a été détectée par écouvillonnage rectal. En raison de la récurrence d'une infection à *Clostridium difficile* résistante à un traitement par metronidazole et fidaxomicine, une première transplantation de microbiote fécal (provenant de la mère de la patiente, également donneur de CSH) a alors été réalisée, 3 mois post-greffe de CSH.

Les selles de la mère avaient été au préalable mélangées avec une solution saline de cryopréservation (glycerol + NaCl 0.9% ,10/90 V/V). L'homogénéisation a été réalisée dans 500ml pour 50-100g de selles. La suspension obtenue a été filtrée en utilisant un entonnoir avec des compresses stériles et des récipients stériles pour retirer tout résidu solide. La solution a ensuite été placée dans un sac de lavement, prêt à l'emploi.

### Résultats :

Les symptômes gastro-intestinaux ont nettement diminué et les examens ont mis en évidence la guérison de l'infection à *Clostridium difficile* et l'éradication de l'entérocoque résistant à la vancomycine.

Six mois post-greffe de CSH, la patiente a présenté une pancytopénie associée à un septis dû à une bactérie pathogène, *Klebsiella pneumoniae,* produisant des beta-lactamases à spectre étendu, et un portage de ERV. De plus, la patiente présentait des douleurs abdominales et une diarrhée importante, sans mise en évidence d'une infection par Clostridium ni réaction gastro-intestinale du greffon contre l'hôte.

Une deuxième transplantation de microbiote fécal (TMF) du même donneur (la mère) a été pratiquée sur la patiente afin de traiter la colonisation par l'ERV. Après la TMF, les signes cliniques se sont améliorés et l'ERV est devenu indétectable.

Aucune bactérie multi-résistante (notamment Clostridium ou ERV) n'a été détectée par écouvillonnage rectal 12 mois post-greffe de CSH.

3 ans post-greffe de CSH, la patiente est en rémission complète de sa leucémie aigüe.

### Exemple 2 : Transplantation de microbiote fécal isologue post-greffe de CSH, chez une patiente atteinte de leucémie lymphoblastique aigüe (mode de réalisation non compris dans l'invention revendiquée)

La patiente est une jeune fille de 19 ans atteinte de leucémie lymphoblastique aigüe en rechute après plusieurs cures de chimiothérapie, quand elle reçoit une greffe de CSH de sa mère (haploidentique), avec conditionnement séquentiel avec du Thiotepa, tel que décrit ultérieurement dans Duléry et al, supra.

Suite à cette greffe, elle développe une GVH aigüe (traitée) puis une GVH chronique. Quatre mois post-greffe CSH, elle est détectée positive aux entérocoques résistants à la vancomycine (ERV).

Six et huit mois post-greffe de CSH, elle reçoit une transplantation de microbiote fécal, de sa mère également, l'échantillon étant préparé comme décrit dans l'exemple 1.

Aucune bactérie multi-résistante n'a été détectée par écouvillonnage rectal par la suite.

Les signes de GVH étaient en voie d'amélioration quand la patiente a rechuté de sa leucémie, deux ans post-greffe de CSH.

### Exemple 3 : Transplantation de microbiote fécal isologue pré-greffe de CSH, chez un patient atteint de leucémie aigüe à cellules dendritiques.

Le patient de 47 ans, diagnostiqué d'une leucémie aigüe à cellules dendritiques, a été soumis à une chimiothérapie (combinaison methotrexate, idarubicine et asparaginase en phase d'induction, puis deux phases de consolidation). Ce patient est testé positivement à *Citrobacter freundii,* ce qui peut être une contre-indication à une greffe de CSH.

Il reçoit une transplantation de microbiote fécal provenant de sa sœur HLA-identique, qui résout la colonisation à *Citrobacter freundii,* rendant le patient apte à recevoir une greffe de CSH. La greffe de CSH (de sa sœur également) est réalisée 9 jours plus tard, après un conditionnement incluant Thiotepa, Busulfan et Fludarabine.

Les selles de la sœur ont été préparées pour la transplantation de microbiote fécal comme expliqué dans l'exemple 1.

Le patient n'a pas développé de réaction aigüe du greffon contre l'hôte dans les suites immédiates de l'allogreffe de CSH, et son traitement immunosuppresseur à base de ciclosporine a pu être arrêté 4 mois après l'allogreffe.

Les complications infectieuses bactériennes (dont une infection par un Staphylocoque résistant à la vancomycine (VRSA)) ont été limitées et contrôlées.

Un an post-greffe, le patient est en rémission.

### Exemple 4 : Transplantation de microbiote fécal non-isologue, pré-greffe de CSH, chez un patient atteint de leucémie myéloïde aigüe

Ce patient âgé de 64 ans, atteint d'une leucémie myéloïde aigüe, a d'abord été traité par deux lignes de chimiothérapie.

Pendant les traitements d'induction de la chimiothérapie, ce patient a développé un sepsis sévère à *Pseudomonas aeruginosa.*

Une transplantation de microbiote fécal a été réalisée à partir des selles de sa fille, préparées selon le protocole de l'exemple 1, qui a permis la négativation du germe multirésistant.

Une allogreffe de cellules souches périphériques à partir d'un donneur HLA-identique, a été réalisée 41 jours plus tard, en absence d'épisodes infectieux majeurs, et après un conditionnement par Busulfan et Fludarabine.

Aucune GVH digestive aigüe n'a été observée suite à la greffe.

Le patient est en rémission (2 ans post-greffe de CSH).

### Exemple 5 : Transplantation de microbiote fécal non-isologue, pré-greffe de CSH, chez un patient atteint de leucémie myéloïde aigüe

Ce patient, âgé de 42 ans, était atteint d'une leucémie myéloïde aigüe, réfractaire à la première ligne de chimiothérapie mais répondant à la deuxième ligne de chimiothérapie.

Ce patient montrait une colonisation à *Pseudomonas aeruginosa* multirésistant, sans complications infectieuses systémiques liées, cependant, dans la perspective d'une allogreffe de CSH et de l'état d'immunodépression conséquent, décision a été prise de réaliser une transplantation de microbiote fécale à partir de sa sœur. Cette TMF a été réalisée selon le protocole de l'exemple 1. 46 jours plus tard, et suite à un conditionnement séquentiel avec du Thiotepa, tel que décrit dans Duléry et al, *supra,* ce patient a pu recevoir une greffe de CSH à partir de son frère haplo-identique.

Aucune complication particulière (ni GVH, ni infection) n'a été observée après la greffe. Cependant, ce patient a rechuté de sa leucémie, et est décédé 11 mois après la greffe de CSH.

### Exemple 6 : Transplantation de microbiote fécal non-isologue, pré-greffe de CSH, chez une patiente atteinte de leucémie myéloïde aigüe

Un diagnostic de leucémie myéloïde aigüe à haut risque a été posé chez cette patiente, âgée de 45 ans, traitée par deux lignes de chimiothérapie à l'Ile Maurice.

Elle arrive en France pour une allogreffe de CSH HLA-identique à partir de sa sœur.

Pendant la période d'hospitalisation pour le bilan pré-greffe et pour régler de multiples problèmes de comorbidité cardio-pulmonaire, on détecte, à l'écouvillon rectal, un *Citrobacter freundii* résistant. A noter que la patiente était dans le même temps colonisée par une *Klebsiella pneumoniae* résistante.

Une transplantation de microbiote fécal (un mélange de selles fraîches et de selles congelées selon le protocole décrit dans l'exemple 1, provenant de son mari) est réalisée par voie rectale, qui résout la colonisation à *Citrobacter freundii,* rendant la patiente apte à recevoir une greffe de CSH 16 jours plus tard.

A noter que la patiente n'a pas présenté de complications infectieuses majeures pendant la période d'aplasie post-greffe. A noter aussi que la *Klebsiella Pneumoniae* qui avait été détectable jusqu'à J60 de la greffe, n'a plus été détectée sur les écouvillons rectaux à la suite. La patiente n'a pas développé de GVH suite à la greffe de CSH (suivi à trois mois).

## Revendications

1. Echantillon de microbiote fécal pour une utilisation dans la prévention d'une maladie GVH résultant d'une greffe allogénique de cellules souches hématopoïétiques (CSH) chez un patient receveur, ledit échantillon de microbiote fécal étant administré avant la greffe de CSH.

2. Echantillon de microbiote fécal pour une utilisation selon la revendication 1, ledit échantillon de microbiote fécal et lesdites cellules souches hématopoïétiques provenant d'un même sujet donneur.

3. Echantillon de microbiote fécal, pour une utilisation selon la revendication 1 ou 2, par transplantation de l'échantillon dans le tube digestif du patient receveur avant ladite greffe de CSH, de préférence de 1 semaine à 12 mois, de préférence 1 semaine à 8 mois, de préférence 1 semaine à 6 mois, de préférence encore de 1 semaine à 4 mois, de préférence 1 semaine à 3 mois, ou de 1 à 8 semaines avant ladite greffe de CSH, éventuellement de manière répétée.

4. Echantillon de microbiote fécal, pour une utilisation selon l'une des revendications 1 à 3, le patient receveur souffrant d'un cancer.

5. Echantillon de microbiote fécal pour une utilisation selon l'une quelconque des revendications 1 à 4, ledit patient receveur souffrant d'une hémopathie maligne.

6. Echantillon de microbiote fécal, pour une utilisation selon l'une des revendications 1 à 5, ledit échantillon étant susceptible d'être obtenu par décongélation à partir d'un i) mélange d'un échantillon de selles du sujet donneur avec une solution aqueuse saline comprenant un agent cryoprotectant et/ou un agent de charge, suivi éventuellement d'une ii) filtration, avant iii) congélation pour conservation, les étapes de mélange et de congélation étant de préférence réalisées dans des conditions d'anaérobiose.

7. Echantillon de microbiote fécal, pour une utilisation selon l'une quelconque des revendications 1 à 6, par administration par voie rectale ou par voie orale.

## Patentansprüche

1. Probe eines fäkalen Mikrobiots für eine Verwendung bei der Vorbeugung einer GVH-Krankheit, resultierend aus einer allogenen Transplantation von hämatopoetischen Stammzellen (HSC) bei einem empfangenden Patienten, wobei die Probe eines fäkalen Mikrobiots vor der HSC-Transplantation verabreicht wird.

2. Probe eines fäkalen Mikrobiots für eine Verwendung nach Anspruch 1, wobei die Probe eines fäkalen Mikrobiots und die hämatopoetischen Stammzellen von demselben Spender stammen.

3. Probe eines fäkalen Mikrobiots für eine Verwendung nach Anspruch 1 oder 2 durch Transplantation der Probe in den Verdauungskanal des empfangenden Patienten vor der HSC-Transplantation, vorzugsweise 1 Woche bis 12 Monate, vorzugsweise 1 Woche bis 8 Monate, vorzugsweise 1 Woche bis 6 Monate, noch vorzugsweiser 1 Woche bis 4 Monate, vorzugsweise 1 Woche bis 3 Monate oder 1 bis 8 Wochen vor der HSC-Transplantation, eventuell wiederholt.

4. Probe eines fäkalen Mikrobiots für eine Verwendung nach eine der Ansprüche 1 bis 3, wobei der empfangende Patient an einem Krebs leidet.

5. Probe eines fäkalen Mikrobiots für eine Verwendung nach einem der Ansprüche 1 bis 4, wobei der empfangende Patient an einer malignen Hämopathie leidet.

6. Probe eines fäkalen Mikrobiots für eine Verwendung nach einem der Ansprüche 1 bis 5, wobei die Probe imstande ist, durch Auftauen eines i) Gemischs aus einer Stuhlprobe des Spenders mit einer wässrigen Salzlösung, umfassend ein Gefrierschutzmittel und/oder einen Füllstoff, erhalten zu sein, gefolgt eventuell von einer ii) Filtration vor iii) Gefrieren zwecks Konservierung, wobei die Schritte des Mischens und des Gefrierens vorzugsweise unter anaerobiotischen Bedingungen durchgeführt werden.

7. Probe eines fäkalen Mikrobiots für eine Verwendung nach einem der Ansprüche 1 bis 6 durch Verabreichung auf rektalem Weg oder auf oralem Weg.

## Claims

1. Sample of fecal microbiota for use in the prevention of a GVH disease resulting from an allogenic hematopoietic stem cell (HSC) transplant in a recipient patient, said sample of fecal microbiota being administered before the HSC transplant.

2. Sample of fecal microbiota for use according to claim 1, said sample de fecal microbiota and said hematopoietic stem cells originating from one and the same donor subject.

3. Sample of fecal microbiota for use according to claim 1 or 2, by transplantation of the sample in the alimentary tract of the recipient patient before said HSC transplant, preferably from 1 week to 12 months, preferably 1 week to 8 months, preferably 1 week to 6 months, more preferably from 1 week to 4 months, preferably 1 week to 3 months, or from 1 to 8 weeks before said HSC transplant, optionally repeatedly.

4. Sample of fecal microbiota for use according to one of claims 1 to 3 where the recipient patient is suffering from a cancer.

5. Sample of fecal microbiota for use according to one of claims 1 to 4, where said recipient patient is suffering from a malignant hemopathy.

6. Sample of fecal microbiota for use according to one of claims 1 to 5, said sample being able to be obtained by thawing from i) mixing a stool sample from the donor subject with a saline aqueous solution comprising a cryoprotectant agent and/or a bulking agent, optionally followed by ii) filtration, before iii) freezing for storage, the steps of mixing and freezing preferably being carried out under anaerobic conditions.

7. Sample of fecal microbiota for use according to one of claims 1 to 6, by administration via the rectal route or via the oral route.
